# EUROPÄISCHE PATENTSCHRIFT

(11) **EP 1 381 405 B1**
(45) Veröffentlichungstag und Bekanntmachung des Hinweises auf die Patenterteilung: **18.05.2005**
(21) Anmeldenummer: 02753689.5
(22) Anmeldetag: 07.03.2002
(51) Int. Cl.: A61L 31/18, A61L 31/08

(54) **RADIOAKTIV BESCHICHTETE STENTS**
RADIOACTIVELY COATED STENTS
TUTEURS INTRAVASCULAIRES A REVETEMENT RADIOACTIF

(30) Priorität: 09.03.2001 DE 10112518
(43) Veröffentlichungstag der Anmeldung: 21.01.2004
(73) Patentinhaber: Schering Aktiengesellschaft, 13353 Berlin (DE)
(72) Erfinder: DINKELBORG, Ludger, 13465 Berlin (DE); MUSCHICK, Peter, 16321 Ladeburg (DE); NOLL, Bernhard, 01705 Freital (DE); GÖRNER, Heidemarie, 01157 Dresden (DE)
(86) Internationale Anmeldenummer: PCT/EP2002/002531
(87) Internationale Veröffentlichungsnummer: WO 2002/076524

(56) Entgegenhaltungen:
- EP-A- 0 938 905
- WO-A-01/72371
- DE-C- 19 724 223
- DE-C- 19 724 229
- US-A- 6 077 413
- US-A- 6 129 658

## Beschreibung

Die Erfindung betrifft neue radioaktiv beschichtete Stents sowie Verfahren zu deren Herstellung.

Radioaktiv beschichtete Stents sowie Verfahren zu deren Herstellung wurden bereits in den Patenten DE 197 24 223, DE 197 24 229, DE 197 24 230, US 6 077 413 sowie in der Patentanmeldung WO 98/48851 beschrieben. Die dort beschriebenen Stents werden als Gefäßimplantate zur Verhinderung der Restenose eingesetzt.

Ein unerwünschter Nebeneffekt nach Stentimplantation ist, daß man häufig an den Stentenden nach einer gewissen Zeit eine Restenose beobachtet, die in der Fachwelt als "candy wrapper"-Effekt bezeichnet wird (*Albiero et al. Edge Restenosis After Implantation of High Activity P-32 Radioactive beta-emitting stents, Circulation 2000, 101: 2454-2457*). Das bedeutet, dass im Stentinneren die Restenose zwar verhindert wird, es an den Stentenden jedoch nach einer gewissen Zeit zu einer Restenose des Blutgefäßes kommt. Bisher sind keine Stents bekannt, die zuverlässig die Restenose nicht nur im Inneren des Stents, sondern auch an dessen äußeren Enden verhindern.

Aufgabe der vorliegenden Erfindung ist es daher ein neues, verbessertes und einfacher durchzuführendes Verfahren nach dem radioaktive Stents hergestellt werden können, bereitzustellen, die nach ihrer Implantation den "candy wrapper"-Effekt nicht mehr zeigen, und die die Restenose sowohl innerhalb des Stents als auch an dessen Enden verhindern.

Diese Aufgabe wird durch das Verfahren zur Herstellung der neuen radioaktiven Stents gelöst, wie es in den Patentansprüchen definiert ist.

Überraschend wurde gefunden, dass Stents, welche mit radioaktiven Isotopen beschichtet sind, welche β-Strahlung mit einer Reichweite von mindestens 10 mm und einer maximalen Energie größer als 2 MeV abgeben, in Tierversuchen keine "candy wrapper"-Effekte zeigen und die Restenose sowohl im Inneren des Stents, als auch an dessen Enden zuverlässig verhindern. Ein geeignetes Isotop ist z.B. Re-188, welches eine Reichweite von 11 mm und eine Energie von 2,11 MeV aufweist. Die kurze Halbwertszeit unter 91 Stunden, im Falle des Re-188 von ca. 17 Stunden, trägt ebenfalls dazu bei, dass die Restenose an den Stentenden nicht induziert wird.

Die Herstellung der radioaktiven Stents erfolgt nach neuen, verbesserten Verfahren.

Bei den bekannten Verfahren zur Herstellung radioaktiver Stents wird ein nichtradioaktiver Stent in eine Lösung, die das radioaktive Isotop in ionischer Form enthält, getaucht, und das Isotop wird dann chemisch auf dem Stent abgeschieden.

Es wurde überraschend gefunden, dass die radioaktiven Stents verbesserte Eigenschaften aufweisen, wenn man sie nach der Abscheidung des radioaktiven Metalls einem Sinterungsprozess im Hochvakuum bei 600 bis 1100°C unterzieht. Durch diesen Sinterungsprozess wird die zunächst metastabil aufgetragene Radioaktivität fixiert, so dass man den Stent besser handhaben kann und er die Aktivität nach Implantation nicht verliert. Die abgeschiedene Schicht wird dadurch mechanisch stabil fixiert, so dass sich die Radioaktivität nicht mehr allein durch Waschen in Wasser oder Ethanol entfernen lässt. Außerdem verbessert sich das Aussehen der Oberfläche der Stents von matt-schwarz zu matt-metallisch, was ein Indiz für eine glattere Oberfläche ist.

Bei dem neuen, verbesserten Verfahren wird zunächst ein nichtradioaktiver Stent in eine Lösung, die das radioaktive Isotop, vorzugsweise Re-188 enthält, getaucht. Vorzugsweise wird das Perrhenat in physiologischer Kochsalzlösung bereitgestellt. Anschließend wird zur Lösung Salzsäure (HCl) zugesetzt, so dass im Ergebnis die Markierungslösung 1 M HCl enthält. Das Reaktionsgefäß wird danach dicht verschlossen und einige Minuten auf ca. 100°C erhitzt. Nach dem Abkühlen wird der Stent entnommen und in einem weiteren Verarbeitungsschritt im Hochvakuum (< 10⁻³ Torr) auf 600 bis 1100°C, vorzugsweise auf ca. 950°C, erhitzt.

Das Ausheizen im Hochvakuum (< 10⁻³ Torr) bei ca. 950°C führt zu einer deutlich verbesserten Oberflächenbeschaffenheit der Stents. Findet der Ausheizschritt bei Normaldruck (760 Torr) statt, dann geht bereits während des Ausheizens ein großer Teil der Aktivität (60-80%) verloren. Die Oberfläche der Stents wird sichtbar geschädigt. Findet das Erhitzen in Inertgasatmosphäre (Argon, Stickstoff, Helium) statt, dann zeigen die so behandelten Stents nach Inkubation in physiologischer Kochsalzlösung rotbraune Beläge und die Inkubationslösungen rotbraune Niederschläge.

Die ektronenmikroskopische Aufnahme (500fache Vergrößerung) der Oberflächen der nach dem verbesserten Verfahren markierten Stents zeigen noch eindeutige Korngrenzen, die etwa mit denen der unbehandelten Stents vergleichbar sind. Mit bloßem Auge erscheinen die Oberflächen etwas matter.

Bei einem alternativen Herstellungsverfahren wird zunächst ein nichtradioaktiver Stent in eine Lösung, die das radioaktive Isotop, vorzugsweise Re-188 enthält, getaucht. Vorzugsweise wird das Perrhenat in physiologischer Kochsalzlösung bereitgestellt. Anstelle von Salzsäure werden in diesem alternativen Verfahren andere Säuren wie z. B. Schwefelsäure verwendet. In diesem Fall kann der anschließende Erhitzungsschritt im Vakuum entfallen. Im Anschluss können sich ein Waschschritt z. B. in verdünntem oder unverdünnten Ethanol sowie ein Sterilisationsvorgang z.B. in einem Trockenschrank oder Sterilisator anschließen. Der Sterilisationsvorgang wird bei 180 bis 220°C, vorzugsweise bei ca. 200°C durchgeführt. Bei diesem alternativen Herstellungsverfahren kann der Ausheizungsschritt im Hochvakuum entfallen.

Bei den in schwefelsaurer Lösung markierten Stents ist unter dem Mikroskop (16 fache Vergrößerung) kein abgeschiedener Belag zu erkennen, die Oberfläche ist silbrigglänzend und nur die Korngrenzen heben sich im Vergleich zum unbehandelten Stent hervor. Durch den Sterilisationsvorgang tritt keine signifikante Veränderung der Oberfläche auf.

Als nichtradioaktive Stents werden handelsübliche Stents, wie z.B. der Wallstent, der Palmaz bzw. Palmaz-Schatz-Stent, der Wiktor-Stent, der AVE-Stent, der GFX-Stent, der Multilink-S-tent, der Radius-Stent, der NIR-Stent, der Jomed-Stent, der Angiomed-Stent, der Nitinol-Stent und andere Stents eingesetzt. Bevorzugt sind Metallstents.

Mit den so hergestellten Stents wurden Inkubationsversuche in menschlichem Blut bzw. in physiologischer Kochsalzlösung durchgeführt. Stents, die unter den optimierten Bedingungen markiert wurden, behalten nach 66 h Inkubation in Human-Vollblut bzw. physiologischer Kochsalzlösung bei 37°C noch mehr als 90 % der aufgebrachten Radioaktivität.

Die Analyse der chemischen Zusammensetzung der Stentoberflächen ergab, daß der Markierungsschritt mit einer Anreicherung der Elemente Molybdän, Silizium und Rhenium (gemessen an ¹⁸⁶Re-markierten Stents) an den Stentoberflächen verbunden ist, die wesentliche Bestandteile des Stent-Edelstahls sind.

Teile des nichtradioaktiven Stents können vor dem Eintauchen in die Lösung, welche das radioaktive Isotop enthält, mit Lack beschichtet werden. Es konnte gezeigt werden, daß an den so beschichteten Stellen nach dem Sinterungsprozess keine bzw. nur wenig Radioaktivität gemessen wird. Lackspuren sind nach dem Sinterungsprozess nicht mehr auf dem Stent vorhanden. So gelingt es, die Aktivität der Stents regional unterschiedlich und individuell zu gestalten. Stents, die an den Enden mehr oder weniger Aktivität im Vergleich zu der restlichen Stentfläche aufweisen, können so hergestellt werden ("cold end" oder "hot end" stents).

In einer Schweinestudie mit 16 Schweinen wurden a) zwei Re-186 beschichtete koronare, 16 mm lange, Stents in den Ramus interventricularis anterior (RIVA) und Ramus circumflexus (RCX) der linken Koronararterie und b) zwei Re-186 beschichtete periphere, 58 mm lange, implantiert.
Bei der Nachuntersuchung (Angiographie und Ultraschall) wurde bei den koronaren Stents (Gruppe a) im Gegensatz zur Kontrolle gefunden, daß Re-186 beschichteten Stents an beiden Stentenden neue stenotische Läsionen ("edge or candy wrapper" Effekte) entwickelten, während das primär zu behandelnde Gefäßsegment im Stent selbst (target lesion) frei von einer Restenose war.

Überraschenderweise wurde bei einer weiteren Schweinestudie, die mit Stents gleicher Abmessungen durchgeführt wurde, welche jedoch statt mit Re-186 mit Re-188 beschichtet waren, bei der Nachuntersuchung gefunden, daß die Re-188 beschichtete Stents sowohl die Restenose an den Stentenden als auch die im Stent selbst sowohl in den Herzkranzgefäßen als auch in den peripheren Gefäßen (Gruppen a) und b)) zuverlässig verhindern. Die erfinderische Aufgabe wurde also gelöst.

In den nachfolgenden Beispielen wird die Herstellung der radioaktiven Stents sowie deren Wirksamkeit *in vivo* detailliert beschrieben.

### Beispiele

### Beispiel 1:

Ein Palmaz-Stent (20 mm, Johnson&Johnson) bzw. ein coronarer Wave Stent (16 mm, JoMed) wird mit 1,5 ml Markierungslösung, bestehend aus x ml [¹⁸⁶Re] bzw. [¹⁸⁸Re] Natriumperrhenatlösung, x ml 2 M HCl und y ml 1 M HCl (2x+y = 1,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt, der Stent entnommen und in Ethanol von anhaftender Markierungslösung und leicht ablösbarer Radioaktivität abgespült. Ca. 20 % der zunächst abgeschiedenen Aktivität, diese beträgt je nach Markierungsdauer ca. 40-60 % des in der Markierungslösung vorgelegten [¹⁸⁸Re]/[¹⁸⁸Re] Natriumperrhenats, werden dabei abgelöst. Um diese metastabil aufgetragene Aktivität zu fixieren, müssen die Stent anschließend in eine Quarzampulle überführt und im Hochvakuum (<10⁻³Torr) 15 min bei 950°C erhitzt werden. Beim Erhitzen werden ca. 2-5 % der Radioaktivität vom Stent entfernt. Die Gesamt-Markierungsausbeute beträgt je nach Markierungsdauer ca. 30-45%.

### Beispiel 2:

Ein Palmaz-Stent (58 mm, Johnson&Johnson) bzw. ein peripherer Wave Stent (58 mm, JoMed) wird mit 4,5 ml Markierungslösung, bestehend aus x ml [¹⁸⁶Re] bzw. [¹⁸⁸Re] Natriumperrhenatlösung, x ml 2 M HCl und y ml 1 M HCl (2x+y = 4,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt, der Stent entnommen und in Ethanol von anhaftender Markierungslösung und leicht ablösbarer Radioaktivität abgespült. Ca. 20 % der zunächst abgeschiedenen Aktivität, diese beträgt je nach Markierungsdauer ca. 40-60 % des in der Markierungslösung vorgelegten [¹⁸⁸Re]/[¹⁸⁸Re] Natriumperrhenats, werden dabei abgelöst. Um diese metastabil aufgetragene Aktivität zu fixieren, müssen die Stent anschließend in eine Quarzampulle überführt und im Hochvakuum (<10⁻³Torr) 15 min bei 950°C erhitzt werden. Beim Erhitzen gehen ca. 2-5 % der Radioaktivität vom Stent ab. Die Gesamt-Markierungsausbeute beträgt je nach Markierungsdauer ca. 30-45 %.

### Beispiel 3:

Ein Palmaz-Stent (20 mm, Johnson&Johnson) bzw. ein coronarer Wave Stent (16 mm, JoMed) wird an beiden Enden in einen Lack (Zaponlack) getaucht und jeweils 2-4 mm mit einer Lackschicht versehen. Der Lack wird an der Luft oder im Warmluftstrom getrocknet.
Der so behandelte Stent wird mit 1,5 ml Markierungslösung, bestehend aus x ml [¹⁸⁶Re] bzw. [¹⁸⁸Re] Natriumperrhenatlösung, x ml 2 M HCl und y ml 1 M HCl (2x+y = 1,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt, der Stent entnommen und in Aceton von dem Lack an den Stentenden, anhaftender Markierungslösung und leicht ablösbarer Radioaktivität abgespült. Ca. 20 % der zunächst abgeschiedenen Aktivität, diese beträgt je nach Markierungsdauer ca. 40-60 % des in der Markierungslösung vorgelegten [¹⁸⁸Re]/[¹⁸⁸Re] Natriumperrhenats, werden dabei abgelöst. Um diese metastabil aufgetragene Aktivität zu fixieren, wird der Stent anschließend in eine Quarzampulle überführt und im Hochvakuum (< 10⁻³Torr) 15 min bei 950°C erhitzt. Beim Erhitzen werden ca. 2-5 % der Radioaktivität vom Stent entfernt. Die Gesamt-Markierungsausbeute beträgt je nach Markierungsdauer ca. 30-45%.

### Beispiel 4:

Ein Palmaz-Stent (58 mm, Johnson&Johnson) bzw. ein peripherer Wave Stent (58 mm, JoMed) wird an beiden Enden in einen Lack (Zaponlack) getaucht und jeweils 2-6 mm mit einer Lackschicht versehen. Der Lack wird an der Luft oder im Warmluftstrom getrocknet mit 4,5 ml Markierungslösung, bestehend aus x ml [¹⁸⁶Re] bzw. [¹⁸⁸Re] Natriumperrhenatlösung, x ml 2 M HCl und y ml 1 M HCl (2x+y = 4,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt, der Stent entnommen und in Aceton von dem Lack an den Stentenden, von anhaftender Markierungslösung und leicht ablösbarer Radioaktivität abgespült. Ca. 20 % der zunächst abgeschiedenen Aktivität, diese beträgt je nach Markierungsdauer ca. 40-60 % des in der Markierungslösung vorgelegten [¹⁸⁸Re]/[¹⁸⁸Re] Natriumperrhenats, werden dabei abgelöst. Um diese metastabil aufgetragene Aktivität zu fixieren, müssen die Stent anschließend in eine Quarzampulle überführt und im Hochvakuum (<10⁻³Torr) 15 min bei 950°C erhitzt werden. Beim Erhitzen gehen ca. 2-5 % der Radioaktivität vom Stent ab. Die Gesamt-Markierungsausbeute beträgt je nach Markierungsdauer ca. 30-45%.

### Beispiel 5:

Ein Palmaz-Stent (20 mm, Johnson&Johnson) bzw. ein coronarer Wave Stent (16 mm, JoMed) wird mit 1,5 ml Markierungslösung (steriles Re-Generatoreluat in physiologischer Kochsalzlösung, bestehend aus x ml [¹⁸⁶Re] bzw. [¹⁸⁸Re] Natriumperrhenatlösung, x ml 2 M H₂SO₄ und y ml 1 M H₂SO₄ (2x+y = 1,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt. Der Stent wird entnommen und in ein Reaktionsgefäß, das 2ml wässriges Ethanol (z.B. 50 %ig) enthält, gelegt. Eventuell kann in einem Ultraschallbad leicht ablösbare Radioaktivität vom Stent abgespült werden. Ca. 30 - 50 % der zunächst abgeschiedenen Aktivität werden dabei abgelöst. Die Markierungsausbeute beträgt je nach Markierungsdauer ca. 10 % des in der Markierungslösung vorgelegten [¹⁸⁸Re]/[¹⁸⁸Re] Natriumperrhenats. Der Stent wird dann in einem Trockenschrank/Sterilisator auf ca. 200 C erhitzt und liegt anschließend in sterilisierter Form vor.

### Beispiel 6:

Ein Palmaz-Stent (58 mm, Johnson&Johnson) bzw. ein peripherer Wave Stent (58 mm, JoMed) wird mit 4,5 ml Markierungslösung, bestehend aus x ml [¹⁸⁶Re] bzw. [¹⁸⁸Re] Natriumperrhenatlösung, x ml 2 M H₂SO₄ und y ml 1 M H₂SO₄ (2x+y = 4,5 ml), überschichtet. Das Reaktionsgefäß wird dicht verschlossen und 10-15 min bei 100°C erhitzt. Danach wird das Reaktionsgefäß im Eisbad auf Zimmertemperatur abgekühlt.
Der Stent wird entnommen und in ein Reaktionsgefäß, das 2ml wässrigem Ethanol (z.B. 50 %ig) enthält, gelegt. In einem Ultraschallbad wird leicht ablösbare Radioaktivität vom Stent abgespült. Ca. 30 - 50 % der zunächst abgeschiedenen Aktivität werden dabei abgelöst. Die Markierungsausbeute beträgt je nach Markierungsdauer ca. 10 % des in der Markierungslösung vorgelegten [¹⁸⁸Re]/[¹⁸⁸Re] Natriumperrhenats. Der Stent wird dann in einem Trockenschrank/Sterilisator auf ca. 200 C erhitzt und liegt anschließend in sterilisierter Form vor.

### Beispiel 7:

Re-186 markierte Stents am Tiermodell Schwein
- **Tiere:**: Göttinger Minischweine, männl., ca. 30kg, n = 17
- **Narkose:**: Einleitung der Narkose: Ketamin 10mg/kg, Stresnil 4mg/kg, Ketamin 10mg/kg i.m. und Ketamin 200mg, Valium 5mg i.v.
Narkose während der OP: Fentanyl/ Droperidol und Lachgas/Sauerstoff 3:1 (0.8/2.4ml) mit Enflurane 1,5-2%
- **Prämedikation:**: 1d vor der OP 500mg Aspirin p.o. und 300mg Clopidrogel p.o.
- **Medikation:**: Am Tag der OP: 5ml Aspisol (500mg)i.v., 1ml (5000 IE) Liquemin i.a., 3ml (150µg) Nitroglycerin i.a., 1.7ml Tardomyocel i.m.
An weiteren Tagen bis zur Tötung: 1xtäglich 75mg Clopidrogel und 100mg ASS p.o.
- **Stents:**: a) Coronare Jomed-Stents 16mm auf 3.5mm dilatiert, beschichtet mit Re-186 nach oben beschriebenem Verfahren mit
   Sinterungsprozeß:
   für RIVA: 10.9 ± 1.58 MBq (n = 9)
   für RCX : 18.1 ± 1.6 MBq (n = 8)
b) Periphere Jomed-Stents 58mm auf 7mm dilatiert, beschichtet mit Re-186 nach oben beschriebenem Verfahren mit
   Sinterungsprozeß:
   für linke Carotiden: 77.5 ± 10.4 MBq (n = 9)
   für rechte Carotiden: 136.8 ± 4.7 MBq (n = 8)
c) Unbehandelte coronare RIVA(n = 8)/ RCX(n = 8) und periphere (n = 12) Stents als Kontrolle

### Versuchsdurchführung:

Die Tiere werden mittels einer i.m. Narkose annarkotisiert. Danach wird ein venöser Zugang in eine Ohrvene gelegt und über diesen Zugang die Narkose intravenös eingestellt.
Der Tubus (Gr.10, Fa. Rüsch) wird mit einem Gleitmittel (Meaverin®Gel) benetzt und mit Hilfe eines Zungenspatels (Laryngoskop) in die Luftröhre eingesetzt und mit dem Ballon des Tubus fixiert.
Die Innenschenkel der Hinterläufe werden rasiert, das Tier mit einem sterilen OP-Tuch abgedeckt und die rasierten Stellen mit einer jodhaltigen Tinktur (Braunol 2000) desinfiziert.
Mit Hilfe einer Punktionsnadel, unter Ultraschallkontrolle, wird die Arteria femoralis punktiert.
Über einen Führungsdraht wird die Schleuse (Fa. Cordis F8-Avanti Einführbesteck) eingeführt und fixiert. Um eine Blutgerinnselbildung zu verhindern, werden 100 IE/kg Liquemin intraarteriell gegeben.

Über die Schleuse wird ein Führungskatheter vorgeschoben, um den Ballonkatheter unbeschädigt vorschieben zu können.
Unter Röntgenkontrolle wird die rechte und linke Carotide mittels Ballonkatheter Gr. 8 durch dreimaliges Aufdrücken auf 8 bar geschädigt. Danach werden die Stents mit Hilfe eines Ballonkatheters Gr. 7 gesetzt und der Katheter auf 8 bar für 30 s aufgedrückt. Anschließend erfolgt eine Kontrollangiographie.
Die coronaren Stents werden jeweils ohne Vordilatation der Gefäße in den Ramus circumflexus (RCX) und Ramus intraventricularis anterior (RIVA) mittels eines Ballonkatheters Gr. 3 und Dilatation auf 10 bar für 30 s unter Röntgenkontrolle gesetzt. Anschließend erfolgt eine Kontrollangiographie. Bei der Arbeit an den Coronarien wird Nitroglycerin nach Bedarf bei auftretenden Spasmus gegeben.
Nach Versuchsende wird die Schleuse gezogen und ein Druckverband angelegt. Es werden 500mg Aspisol und 1.7ml Tardomyocel intramusculär verabreicht.
Bis zur spontanen Eigenatmung wird das Tier mit Raumluft beatmet. Danach wird der Tubus gezogen.
Die Stabilität der Re-186-Beschichtung wird mittels Gammakamera (Elscint SP-4 HR, Energie:63keV/ 136keV) nach OP-Ende und 2h post Stentapplikation untersucht. Die Tiere werden für diese Zeit mit Ketamin i.v. nach Bedarf in Narkose gehalten.
Das Aufwachen der Tiere erfolgt unter Beobachtung im eigenen Käfig mit Futter und Wasser.

### Nachuntersuchung: 8 Wochen nach Stentimplantation

Die Tiere werden mittels einer i.m. Narkose annarkotisiert. Danach wird ein venöser Zugang in eine Ohrvene gelegt und über diesen Zugang die Narkose intravenös eingestellt.
Der Tubus (Gr.10, Fa.Rüsch) wird mit einem Gleitmittel (Meaverin®Gel) benetzt und mit Hilfe eines Zungenspatels (Laryngoskop) in die Luftröhre eingesetzt und mit dem Ballon des Tubus fixiert.
Die Innenschenkel der Hinterläufe werden rasiert, das Tier mit einem sterilen OP-Tuch abgedeckt und die rasierten Stellen mit einer jodhaltigen Tinktur desinfiziert.

Mit Hilfe einer Punktionsnadel und unter Ultraschallkontrolle wird die Arteria femoralis punktiert.
Über einen Führungsdraht wird die Schleuse (Fa. Cordis F8-Avanti Einführbesteck) eingeführt und fixiert.
Um eine Blutgerinnselbildung zu verhindern, werden 100 IE/kg Liquemin intraarteriell gegeben.
Ein Führungskatheter wird in die gestenteten Gefäße vorgeschoben und die Gefäße mittels Angiographie (Ultravist-370 Fa. Schering) und IVUS Ultraschalluntersuchung dargestellt. Bei der Untersuchung an den Herzkranzgefäßen wird Nitroglycerin nach Bedarf bei auftretenden Spasmus gegeben.
Nach Versuchsende wird die Schleuse gezogen und ein Druckverband angelegt. Bis zur spontanen Eigenatmung wird das Tier mit Raumluft beatmet. Danach wird der Tubus gezogen.
Das Aufwachen der Tiere erfolgt unter Beobachtung im eigenen Käfig mit Futter und Wasser.

### Stententnahme: 6 Monate nach Stentimplantation

Die Tiere werden mittels einer i.m. Narkose annarkotisiert. Danach wird ein venöser Zugang in eine Ohrvene gelegt und über diesen Zugang die Narkose intravenös eingestellt.
Zusätzlich wird Narcoren (4ml) und Fentanyl/ Droperidol (0.1/ 5mg) i.v verabreicht. Zur Verhinderung von Thrombenbildung, werden 1000 IE Liquemin i.v. gegeben.
Die Tiere werden mit 20ml KCL (7.45%ig) i.v. getötet.
Danach erfolgt die Entnahme der gestenteten Gefäßstücke, welche gespült und mit formalinhaltiger Fixierlösung (2 % Paraformaldehydlösung in Cacodylat Puffer) fixiert werden.
Anschließend werden histologische Untersuchungen der Gefäße im Stentbereich und deren Beurteilung durchgeführt.

### Ergebnisse:

Die in-stent Restenose wurde sowohl in beiden Bereichen der linken Coronarie als auch in den Karotiden signifikant unterdrückt. Stellvertretend für jedes Verumtier können bei den eingesetzten Dosierungen von Re-186 Randeffekte (candy-wrapper) in allen untersuchten Gefäßprovenienzen beobachtet werden.

### Beispiel 8:

Schweinestudie Re-186 cold-end Stents und niedrigere Dosierung
- **Tiere:**: Göttinger Minischweine, männl., ca. 30kg, n = 4 (gültig für Beispiel 8 und 9).
- **Narkose:**: Fentanyl/ Droperidol und Lachgas/ Sauerstoff 3:1 (0.8/2.4ml) mit Enflurane 1,5-2%
- **Prämedikation:**: 1d vor der OP 500mg Aspirin p.o. und 300mg Clopidrogel p.o.
- **Medikation:**: Am Tag der OP: 5ml Aspisol (500mg)i.v., 1ml (5000 IE) Liquemin i.a., 3ml (150µg) Nitroglycerin, 1.7ml Tardomyocel An weiteren Tagen bis zur Tötung: 1xtäglich 75mg Chlopidrogel und 100mg ASS p.o.
- **Stents:**: a) Coronare Jomed-Stents 16mm auf 3.5mm dilatiert, beschichtet nach oben beschriebenem Verfahren mit Sinterungsprozeß: Re-186komplett: 0.8/1.9 MBq (RCX/RIVA)
b) Periphere Jomed-Stents 58mm auf 7mm dilatiert, beschichtet nach oben beschriebenem Verfahren mit Sinterungsprozeß: Re-186cold end: 12/27 MBq (re A.c./ re A.c.)*
   komplett: 11/28 MBq (li A.c./ li A.c.)*
   *rechte Arteria carotis communis (re A.c.), linke Arteria carotis communis (li A.c.)
c) Die Kontrolle (n=1, li A.c.) ist mit kaltem Rhenium. (Verfahren wie oben beschrieben, mit Sinterungsprozeß) beschichtet (gültig für Beispiel 8 und 9).

Cold end Stents besitzen auf jeder Seite ein 4mm langes Stück ohne Aktivität (Herstellung siehe Beispiel 3 und 4).

### Durchführung: siehe Beispiel 7

### Nachuntersuchung: 4 Wochen nach Stentimplantation

siehe Beispiel 7

### Ergebnis:

Auch bei diesen reduzierten Re-186-Dosierungen konnte im Gegensatz zu den Kontrollen keine in-stent Restenose sowohl in den peripheren als auch in den coronaren Arterien beobachtet werden. In den Karotiden konnten auch keine Randeffekte beobachtet werden. In der linken Coronararterie traten jedoch auch bei dieser erniedrigten Dosierung Randeffekte auf. Bei den "cold end stents" wurden Randeffekte im Bereich der Stentenden beobachtet. Die mit dem beschriebenen Verfahren markierten Stents, die ohne Radioaktivität eingesetzt wurden, zeigen im Vergleich zur Kontrolle (siehe Beispiel 7) keine erhöhte Restenosebildung.

### Beispiel 9:

Schweinestudie Re-188
- **Tiere:**: Göttinger Minischweine, männl., ca. 30kg, n = 4 (gültig für Beispiel 8 und 9).
- **Narkose:**: Fentanyl/ Droperidol und Lachgas/ Sauerstoff 3:1 (0.8/2.4ml) mit Enflurane 1,5-2%
- **Prämedikation:**: 1d vor der OP 500mg Aspirin p.o. und 300mg Clopidrogel p.o.
- **Medikation:**: Am Tag der OP: 5ml Aspisol (500mg)i.v., 1ml (5000 IE) Liquemin i.a., 3ml (150µg) Nitroglycerin, 1.7ml Tardomyocel An weiteren Tagen bis zur Tötung: 1xtäglich 75mg Chlopidrogel und 100mg ASS p.o.
- **Stents:**: a) Coronare Jomed-Stents 16mm auf 3.5mm dilatiert, beschichtet nach oben beschriebenem Verfahren mit Sinterungsprozeß: Re-188cold end: 0.8/1.7 MBq (RIVA/RIVA)
   komplett: 1.5/3 MBq (RCX/RCX)
b) Periphere Jomed-Stents 58mm auf 7mm dilatiert, beschichtet nach oben beschriebenem Verfahren mit Sinterungsprozeß:
   Re-188cold end: 18 MBq ((li A.c.)
   komplett: 21 MBq (re A.c.)
c) Die Kontrolle (n=1, li A.c.) ist mit kaltem Rhenium beschichtet (gültig für Beispiel 8 und 9).

Cold end Stents besitzen auf jeder Seite ein 4mm langes Stück ohne Aktivität

### Durchführung: siehe Beispiel 7

Nachuntersuchung: 4 Wochen nach Stentimplantation, siehe Beispiel 7

### Ergebnis:

Bei allen untersuchten Re-188 markierten Stents, sind weder eine in-stent Restenose noch Randeffekte zu beobachten.

## Patentansprüche

1. Verfahren zur Herstellung radioaktiver Stents, welche β-Strahlung mit einer Reichweite von mindestens 10 mm und einer Energie größer als 2 MeV abgeben, bei dem ein nichtradioaktiver Stent in eine Lösung, die das radioaktive Isotop in ionischer Form enthält, getaucht wird, und das Isotop dann chemisch auf dem Stent abgeschieden wird, **dadurch gekennzeichnet, dass** der radioaktive Stent anschließend einem Sinterungsprozess im Hochvakuum, welches <10⁻³ Torr ist, bei 600 bis 1100°C unterzogen wird.

2. Verfahren gemäß Anspruch 1, **dadurch gekennzeichnet, dass** der Sinterungsprozess bei 950°C durchgeführt wird.

3. Verfahren gemäß Anspruch 1, **dadurch gekennzeichnet, dass** das radioaktive Isotop eine Halbwertszeit von weniger als 91 Stunden hat.

4. Verfahren gemäß Anspruch 1, **dadurch gekennzeichnet, dass** das radioaktive Isotop eine Halbwertszeit von 17 Stunden oder weniger hat.

5. Verfahren gemäß einem der Ansprüche 1-4, **dadurch gekennzeichnet, dass** das radioaktive Isotop Re-188 ist.

6. Verfahren gemäß Anspruch 1, **dadurch gekennzeichnet, dass** die Lösung eine Säure, insbesondere Schwefel- oder Salzsäure, enthält.

7. Verfahren gemäß Anspruch 6, **dadurch gekennzeichnet, dass** die Lösung zusätzlich Kochsalz enthält.

8. Verfahren gemäß einem der Ansprüche 6 oder 7, **dadurch gekennzeichnet, dass** der Stent in einem weiteren Verarbeitungsschritt in einem Trockenschrank oder einem Sterilisator auf 180 bis 220°C erhitzt wird.

9. Verfahren gemäß einem der Ansprüche 6 bis 8, **dadurch gekennzeichnet, dass** das radioaktive Isotop β-Strahlung mit einer Reichweite von mindestens 10 mm und einer Energie größer als 2 MeV abgibt.

10. Verfahren gemäß einem der Ansprüche 6 bis 9, **dadurch gekennzeichnet, dass** das radioaktive Isotop eine Halbwertszeit von weniger als 91 Stunden hat.

11. Verfahren gemäß einem der Ansprüche 6 bis 9, **dadurch gekennzeichnet, dass** das radioaktive Isotop eine Halbwertszeit von 17 Stunden oder weniger hat.

12. Verfahren gemäß einem der Ansprüche 6 bis 9, **dadurch gekennzeichnet, dass** das radioaktive Isotop Re-188 ist.

13. Radioaktive Stents, herstellbar nach einem Verfahren gemäß einem der Ansprüche 1 bis 12.

14. Radioaktive Stents gemäß Anspruch 13, **dadurch gekennzeichnet, dass** die Stentenden eine andere Radioaktivität als die Stentmitte aufweisen.

## Claims

1. Process for the production of radioactive stents that release β-radiation within a radius of at least 10 mm and with an energy of greater than 2 MeV, in which a non-radioactive stent is immersed in a solution that contains the radioactive isotope in ionic form, and the isotope is then chemically deposited on the stent, **characterized in that** the radioactive stent then is subjected to a sintering process under high vacuum, which is <10⁻³ Torr, at 600 to 1100°C.

2. Process according to Claim 1, **characterized in that** the sintering process is performed at 950°C.

3. Process according to Claim 1, **characterized in that** the radioactive isotope has a half-life of less than 91 hours.

4. Process according to Claim 1, **characterized in that** the radioactive isotope has a half-life of 17 hours or less.

5. Process according to one of Claims 1-4, **characterized in that** the radioactive isotope is Re-188.

6. Process according to Claim 1, **characterized in that** the solution contains an acid, especially sulphuric acid or hydrochloric acid.

7. Process according to Claim 6, **characterized in that** the solution contains additionally common salt.

8. Process according to one of Claims 6 or 7, **characterized in that** the stent is heated in a further processing step in a drying oven or a sterilizer to 180 to 220°C.

9. Process according to one of Claims 6 to 8, **characterized in that** the radioactive isotope releases β-radiation within a radius of at least 10 mm and with an energy of greater than 2 MeV.

10. Process according to one of Claims 6 to 9, **characterized in that** the radioactive isotope has a half-life of less than 91 hours.

11. Process according to one of Claims 6 to 9, **characterized in that** the radioactive isotope has a half-life of 17 hours or less.

12. Process according to one of Claims 6 to 9, **characterized in that** the radioactive isotope is Re-188.

13. Radioactive stents that can be produced according to a process according to one of Claims 1 to 12.

14. Radioactive stents according to Claim 13, **characterized in that** the stent ends have a different radioactivity from that of the middle of the stent.

## Revendications

1. Procédé de fabrication de stents radioactifs qui émettent un rayonnement β dont la portée est supérieure ou égale à 10 mm et dont l'énergie est supérieure à 2 MeV, dans lequel un stent non radioactif est plongé dans une solution qui contient l'isotope radioactif sous forme ionique et dans lequel l'isotope est alors déposé chimiquement sur le stent, **caractérisé en ce que** le stent radioactif est ensuite fritté sous vide poussé de < 10⁻³ Torr à une température comprise entre 600 et 1100 °C.

2. Procédé selon la revendication 1, **caractérisé en ce que** le frittage est effectué à 950 °C.

3. Procédé selon la revendication 1, **caractérisé en ce que** l'isotope radioactif a une demi-vie inférieure à 91 heures.

4. Procédé selon la revendication 1, **caractérisé en ce que** l'isotope radioactif a une demi-vie inférieure ou égale à 17 heures.

5. Procédé selon l'une des revendications 1 à 4, **caractérisé en ce que** l'isotope radioactif est le Re-188.

6. Procédé selon la revendication 1, **caractérisé en ce que** la solution contient un acide et en particulier de l'acide chlorhydrique ou de l'acide sulfurique.

7. Procédé selon la revendication 6, **caractérisé en ce que** la solution contient en outre du sel de cuisine.

8. Procédé selon l'une des revendications 6 ou 7, **caractérisé en ce que**, lors d'une étape supplémentaire de traitement, le stent est chauffé à une température comprise entre 180 et 220°C dans une boîte sèche ou dans un stérilisateur.

9. Procédé selon l'une des revendications 6 à 8, **caractérisé en ce que** l'isotope radioactif émet un rayonnement β dont la portée est d'au moins 10 mm et dont l'énergie est supérieure à 2 MeV.

10. Procédé selon l'une des revendications 6 à 9, **caractérisé en ce que** l'isotope radioactif a une demi-vie inférieure à 91 heures.

11. Procédé selon l'une des revendications 6 à 9, **caractérisé en ce que** l'isotope radioactif a une demi-vie inférieure ou égale à 17 heures.

12. Procédé selon l'une des revendications 6 à 9, **caractérisé en ce que** l'isotope radioactif est le Re-188.

13. Stent radioactif qui peut être fabriqué selon un procédé selon l'une des revendications 1 à 12.

14. Stent radioactif selon la revendication 13, **caractérisé en ce que** ses extrémités ont une radioactivité différente de celle de son milieu.
